(19) Europäisches Patentamt
European Patent Office
Office européen des brevets

(11) **EP 1 471 353 B1**

(12) **EUROPÄISCHE PATENTSCHRIFT**

(45) Veröffentlichungstag und Bekanntmachung des Hinweises auf die Patenterteilung:
**17.06.2009 Patentblatt 2009/25**

(51) Int Cl.:
***G01N 33/49*** *(2006.01)*

(21) Anmeldenummer: **04017857.6**

(22) Anmeldetag: **24.01.2001**

(54) **Vorrichtung und Verfahren zur Erfassung von Gerinnungsfunktionen der globalen, insbesondere der primären Hämostase**

Device and method for detecting the coagulation functions of global, especially primary hemostasis

Dispositif et procédé pour determiner les fonctions de coagulation de l'hémostase globale, notamment primaire

(84) Benannte Vertragsstaaten:
**AT BE CH CY DE DK ES FI FR GB GR IE IT LI LU MC NL PT SE TR**

(30) Priorität: **25.01.2000 DE 10003093**

(43) Veröffentlichungstag der Anmeldung:
**27.10.2004 Patentblatt 2004/44**

(62) Dokumentnummer(n) der früheren Anmeldung(en) nach Art. 76 EPÜ:
**01913766.0 / 1 255 991**

(73) Patentinhaber:
• **Freiherr von der Goltz, Volker**
**D-83370 Seeon (DE)**
• **Kratzer, Michael, Dr.**
**D-80799 München (DE)**

(72) Erfinder:
• **Volker Freiherr von der Goltz**
**83370 Seeon (DE)**
• **Kratzer, Michael, Dr.**
**80799 München (DE)**

(74) Vertreter: **Eisenführ, Speiser & Partner**
**Patentanwälte Rechtsanwälte**
**Postfach 31 02 60**
**80102 München (DE)**

(56) Entgegenhaltungen:
EP-A- 0 138 190          EP-A- 0 635 720
DE-A1- 19 617 407        US-A- 5 275 953
US-A- 5 339 830          US-A- 5 599 718
US-A- 5 662 107          US-A- 6 004 819

## Beschreibung

Die Erfindung betrifft eine Vorrichtung nach dem Oberbegriff des Patentanspruches 1 und ein Verfahren nach dem Oberbegriff des Patentanspruches 4.

## [Stand der Technik]

Bei einem derartigen Verfahren und einer derartigen Vorrichtung, welche aus der DE 196 17 407 A1 bekannt sind, wird aus einem Vorratsgefäß mittels einer Kolben-Zylinder-Anordnung ein Volumenstrom eines zu untersuchenden Blutes durch einen als Apertur ausgebildeten Strömungsweg einer Reaktionseinrichtung transportiert. Durch Aggregation und/oder Koagulation von Blutbestandteilen, insbesondere Blutplättchen, ergibt sich eine zunehmende Verstopfung der Apertur. Der sich dabei ausbildende Druckabfall an der Ausgangsseite der Apertur wird in einem vom zu untersuchenden Blut freien Druckmessraum über eine Zuleitung zu einem Drucksensor erfaßt und gemessen. Der Druckmessraum befindet sich unterhalb einer den Arbeitsraum der Kolben-Zylinder-Anordnung begrenzenden Kolbenfläche des vertikal nach oben bewegten Kolbens. Das zu untersuchende Blut wird dabei aus einem unterhalb der Apertur befindlichen Vorratsraum nach oben befördert. Der Druckmessraum befindet sich zwischen der Oberfläche des durch die Apertur geförderten Blutes und der Unterseite des Kolbens. Über die durch den Kolben geführte Zuleitung, welche mit dem Drucksensor verbunden ist, wird der Druck im Druckmessraum abgetastet und gemessen.

Ferner ist aus der EP 0 635 720 A1 ein Verfahren und eine Vorrichtung bekannt, bei welchen die Anlagerung bzw. Aggregation der Blutplättchen unter bestimmten Fließbedingungen initiiert wird. Durch eine Rotationsbewegung, welche das zu untersuchende Blut gegenüber einer Oberfläche ausführt, treten an der Oberfläche Scherkräfte auf. Blutplättchen lagern sich dabei am Boden des Behälters, in welchem das zu untersuchende Blut sich befindet, an. Anschließend erfolgt eine Auswertung der abgelagerten Blutteilchen durch Elektronenmikroskopabtastung oder optischer Bildanalyse oder dergleichen.

Ferner ist es aus der EP 0 138 190 B1 bekannt, daß in einer Membranöffnung, durch welche eine Blutprobe hindurchtransportiert wird, eine Aggregation von Blutplättchen durch Scherkräfte in der Öffnung (Apertur) ausgelöst werden kann. Zur Messung der Aggregation der Blutplättchen wird die Blutprobe über der Meßöffnung unter Druck gesetzt und die Zeit gemessen in der eine bestimmte Verringerung der Meßöffnung durch Aggregation von Blutplättchen eingetreten ist.

Ferner ist aus einem Aufsatz von Hubert Poliwoda et al, "Das Thrombometer: Seine Bedeutung als Globaltest zur Beurteilung der Thrombozytenfunktion" (Klin. Lab. 6/95, Seiten 457 bis 464) eine Vorrichtung bekannt, mit der die Reaktion von Thrombozyten ohne unphysiologische, mechanische oder chemische Einflüsse untersucht werden kann. Hierzu wird mittels einer Kanüle, an welche die Reaktionseinrichtung angeschlossen ist, Vollblut aus der Vene eines Patienten entnommen. Die Reaktionseinrichtung enthält ein Kollagen-Plättchen mit einer exakt gebohrten Öffnung mit einem Durchmesser von 0,5 mm. Durch diese Öffnung strömt das entnommene Blut mit einer Geschwindigkeit von 8 cm/s, wobei jedes Blutplättchen die Öffnung im Kollagen innerhalb von etwa 50 ms durchläuft. Ein konstanter Durchfluss wird mittels einer mechanisch betriebenen Pumpe erzwungen, welche mit der Reaktionseinrichtung verbunden ist. Das Blut wird mit einer Geschwindigkeit von 0,94 ml/min aspiriert. Wenn die Thrombozyten die Kollagenöffnung passieren, werden sie festgehalten und verschließen zunehmend das Bohrloch im Kanal der Reaktionseinrichtung. Hierbei wird die Zeit bestimmt, in der der Saugdruck von 50 auf 150 mbar ansteigt. Ferner kann zwischen der Kanüle und der Reaktionseinrichtung eine Vorrichtung plaziert sein, durch die kontinuierlich die Lösung einer Substanz zugeführt wird, deren Einfluß auf die Thrombozytenfunktion an Kollagen geprüft werden kann. Die Druckmessung erfolgt dabei in dem Saugschlauch, welcher mit 0,9 %-ziger NaCl-Lösung gefüllt ist.

Aus der US 5,662,107 ist eine Messvorrichtung bekannt, mit welcher in vitro eine Thrombusbildung bei simulierten Invivo-Bedingungen gemessen wird. Hierzu wird Blut mit konstanter Fließrate durch einen Kanal gepumpt, der aus einem die Thrombusbildung fördernden Material besteht oder mit einem solchen Material beschichtet ist. Dabei wird stromaufwärts und stromabwärts von der thrombusbildenden Einheit eine Druckmessung durchgeführt und der Druckunterschied als Tendenz zur Thrombusbildung ausgewertet. Dabei wird auf die Bedeutung der Scherrate für die Anlagerung von Plättchen und die Aktivierung von Koagulation hingewiesen. Hierbei wird jedoch außer Acht gelassen, dass bei zunehmender Anlagerung von Plättchen die Scherrate unkontrolliert ansteigt, wenn die Fließrate beibehalten wird.

## [Aufgabe der Erfindung]

Aufgabe der Erfindung ist es eine Vorrichtung und ein Verfahren der eingangs genannten Art zu schaffen, bei denen mit geringem Meßaufwand genaue und reproduzierbare Meßergebnisse erreicht werden.

Diese Aufgabe wird bei der Vorrichtung durch die kennzeichnenden Merkmale des Patentanspruches 1 und beim Verfahren durch die kennzeichnenden Merkmale des Patentanspruches 4 gelöst.

Hierdurch wird mittels einfacher und wartungsarmer Technik, eine sehr preisgünstige Meßvorrichtung geschaffen, was ebenso für die Meßeinsätze gelten kann. Die Vorrichtung bietet die Vorraussetzung zur preiswerten Gestaltung eines mehrkanaligen Automaten für den Einsatz in Großlabors sowie für ein kleines Gerät mit z.B. einem Messkanal, zum Einsatz unter erschwerten Bedingungen im patientennahen Bereich (Point of Care). Das Verfahren ermöglicht den Meßablauf unter kontrollierten Scherbedingungen in der sich verändernden Reaktionsöffnung vorzunehmen und damit neue wichtige Diagnoseansätze zu bilden.

Für die Messungen der Gerinnungsfunktionen der globalen, insbesondere der primären Hämostase kann Vollblut oder plättchenreiches Plasma verwendet werden. Als Antikoagulanzien können Natriumcitrat, Hirudin und andere Substanzen zum Einsatz kommen. Zur Auslösung der Gerinnung können sich Aktivatoren wie z.B. Kollagen, Adenosindiphosphat, Thrombin und weitere nachstehend aufgeführte Substanzen, auf den Begrenzungsflächen der Reaktionsöffnungen befinden oder können der Blutprobe vor oder während der Messung zugegeben werden.

Die Begrenzungsfläche einer oder mehrerer Reaktionsöffnungen, an denen Blutbestandteile unter Einwirkung von Scherkräften gegebenenfalls anlagern oder reagieren, können z.B. aus hydrophilem, gegebenenfalls auch hydrophobem Kunststoff, Glas, nicht porösen bioaktiven Folien/Membranen, Kollagenmembranen bestehen und sind weiterhin zusätzlich für unterschiedliche Fragestellungen der Gerinnungs- bzw. Plättchenreaktion reaktiv gestaltet, in dem sie bioaktiv beschichtet sind z. B. mit Thrombin oder Batroxobin oder einer extrazellulären Matrix (ECM) oder mit Kollagen (auch natürliches rekombinantes Kollagen oder gereinigte Kollagen-Subtypen) oder synthetischen Peptiden mit kollagenähnlichen Aminosäuresequenzen oder Laminin, Fibronektin, bevorzugt mit Thrombospondin, Erythrozyten und/oder Leukozyten, bevorzugt der Blutgruppe Null oder von-Willebrand-Faktor oder einer Mixtur von Kollagen (wie oben) oder synthetischen Peptiden mit Substanzen wie Adenosindiphosophat (ADP), Adrenalin, Fibronektin, Thrombospondin und/oder anderen die Gerinnungsreaktion induzierenden Agenzien (EP 0 316 599 A2) (EP 0 111 942) (US 5,854,067; US 5,662,107).

In einem Blutgefäß verhält sich die Fließgeschwindigkeit des Blutes antiproportional zum Radius des Blutgefäßes und ist an der Gefäßwand geringer als im Gefäßzentrum. Der Geschwindigkeitsunterschied von angrenzenden Flüssigkeitsschichten, die parallel aneinander vorbei fließen, produziert zwischen diesen Schichten einen Schereffekt. Er ist am größten an der Gefäßwand und nimmt in Richtung zum Gefäßzentrum ab. Die lokale Scherrate, die dem Geschwindigkeitsgradienten zwischen zwei angrenzenden vorbeifließenden Flüssigkeitsschichten entspricht, beeinflusst den Scherstress und verhält sich direkt proportional zu ihm. Entsprechend herrschen in unterschiedlichen Gefäßtypen an der Oberfläche der Gefäßwände verschiedene Scherraten. Physiologische Scherraten sind in großen Venen $<100$ s$^{-1}$. Entsprechend dem Durchmesser von Arterien, variieren dort die Wandscherraten zwischen 100 - 1000 s$^{-1}$ und erreichen in Arteriolen ungefähr 1500 s$^{-1}$· In den Koronararterien liegt die durchschnittliche Scherrate bei zirka 650 s$^{-1}$. Extrem hohe Scherraten von ca. 3000 s$^{-1}$ bis max. 40 000 s$^{-1}$ existieren in arteriosklerotisch verengten Gefäßen. Je nach Höhe des Scherstresses verändern sich bei bestimmten Zellenarten, insbesondere bei Thrombozyten, die äußeren Formen und Reaktionsfähigkeiten sowie das Bindungsverhalten der Membran- bzw. Plasmaproteine. Es ist bekannt, dass normale, insbesondere jedoch aktivierte Blutplättchen, mit zunehmender Steigerung der Scherrate, ebenfalls zunehmend gesteigert z.B. an Kollagenoberflächen'adherieren und anschließend aggregieren (Arteriosklerose). Dagegen können Plättchen mit gehemmter Funktion durch Einwirkung von z.B. ASS (Acetylsalicylsäure) oder durch eine vorhandene von-Willebrand-Erkrankung, mit zunehmender Steigerung der Scherrate, immer weniger an z.B. Kollagenoberflächen adherieren und somit aggregieren (Blutungsneigung). Diese Erkenntnis lässt sich bei der Erfindung zur sensiblen Diagnose von Plättchenfunktionen der primären Hämostase nützen, in dem man kontrolliert einen Blutvolumenstrom in der Weise durch eine mit z.B. Kollagen beschichtete Apertur oder Reaktionsöffnung bewegt, dass dort eine vorgegebene, insbesondere konstante Scherrate in der sich durch Anlagerung und Aggregation von Blutplättchen verkleinernden oder verschließenden Apertur/Reaktionsöffnung beibehalten wird. Der Volumenstrom des zu untersuchenden Blutes durch die Apertur/Reaktionsöffnung kann auch in Abhängigkeit einer beliebigen vorbestimmten Scherraten- bzw. Scherkraftkennlinie eingestellt werden.

Der durch die geregelte Bewegung des Kolbens in der Kolben-Zylinder-Anordnung entstandene Förderstrom erzeugt einen Förderdruck, der sich gemäß dem Strömungswiderstand in der oder den Reaktionsöffnungen aufbaut. Der Blutstrom erzeugt in der oder den Reaktionsöffnungen Scherbedingungen oder Fließbedingungen unter deren Mitwirkung Blutplättchen entsprechend ihrer Funktionsfähigkeit an den Begrenzungsflächen in der Reaktionsöffnung/en die bioaktiv oder anlagerungsfähig gestaltet sind, gegebenenfalls anhaften und aggregieren und dabei den offenen Querschnitt der Reaktionsöffnung verringern oder diese Reaktionsöffnung durch Bildung eines Thrombus ganz verschließen können oder es kommt durch den Einfluss der reaktiv gestalteten Begrenzungsfläche(en) der Reaktionsöffnung (en)oder durch den Einfluss von zugeführten Aktivatoren, zu einer Veränderung der Fließfähigkeit des Blutes, durch Einsetzen der globalen Blutgerinnung, insbesondere durch Veränderung der physikalischen Struktur von polymerisiertem Fibrin und zellulären Komponenten (Plättchen, Erythrozyten, Leukozyten) oder durch einen Anstieg der Kraft, die durch aktivierte Thrombozyten auf das Fibrinnetzwerk ausgeübt wird. Dabei ergibt sich im Förderweg des durch die Reaktionseinrichtung transportierten, zu untersuchenden Blutes ein sich ändernder Druck, der im Druckmessraum wirksam wird, welcher der Reaktionsöffnung vor- oder nachgelagert ist und zur Regelung des geförderten Volumenstroms verwendet wird. Erfindungsgemäß wird der Volumenstrom so eingestellt, dass in Abhängigkeit vom jeweils gemessenen Druck, die Scherrate bzw. Scherkraft, welche an der Reaktionsstelle bzw. in der Reaktionsöffnung wirksam ist, einer vorgegebenen Kennlinie folgt, die vorzugsweise einer konstanten Scherrate/-kraft entspricht oder eines anderen vorgegebenen Scher- oder Fließgeschwindigkeitsverlaufes. Die hierbei gewonnenen Messergebnisse und Auswertungsresultate entsprechen dem tatsächlichen Anlagerungs- und Aggregationsverhalten der Blutplättchen entsprechend der Plättchenreaktion der primären Hämostase oder dem Gerinnungsverhalten der globalen Hämostase. Für die klinische Bewertung können, der dann vorhandene Volumenstrom und/oder die Volumenflussmenge, welche nach Ablauf einer bestimmten vorgegebenen Messzeit durch die Reaktionseinrichtung geflossen ist erfasst werden oder die abgelaufene Zeit und/oder die Volumenflussmenge, wenn der Volumenstrom einen vorbestimmten Wert erreicht hat oder gegen Null geht (DE 35 41 057 A1). Ferner können für die klinische Bewertung bei vorgegebener Volumenflussmenge, die hierfür abgelaufene Zeit und/oder der dann vorhandene Volumenstrom als Plättchenparameter ausgewertet werden.

Des weiteren kann die gemessene Druckveränderung und/oder die erreichte Volumenflussmenge nach einer vorgegebenen Zeit oder die abgelaufene Zeit bei Erreichen eines vorgegebenen Druckwertes und/oder einer Volumenflussmenge als Messparameter für die globale, insbesondere primäre Hämostase verwendet werden.

Ein sehr wichtiger Aspekt für die klinische Akzeptanz eines Verfahrens ist neben der Lieferung von klinisch relevanten Daten, dessen kostengünstige Anwendung durch preiswerte Einmalmesseinsätze und die Möglichkeit mit kleinen Blutprobenmengen die Messungen durchzuführen. Deshalb besteht bei den beschriebenen Reaktionseinrichtungen entsprechender Ausführung die Möglichkeit kleine Blutmengengen so für die Messung zu verwenden, dass unter den beschriebenen Messbedingungen Blut aus dem Vorratsraum über die Reaktionseinrichtung in den Sammelraum und wieder zurück befördert wird, wobei es zu Gerinnungs- bzw. Plättchenreaktionen der globalen, insbesondere der primären Hämostase in der oder den Reaktionsöffnungen kommen kann und zwar solange, bis die parameterbildenden Messgrenzen (Zeit, Volumenmenge, Volumenstrom, Druck, Anlagerungsformation für die optische Auswertung usw.) des jeweiligen Messprogrammes erreicht worden sind, um dann diagnosefähige Ergebnisse bilden zu können, wie zuvor beschrieben.

Verfahren zur Messung der Plättchenreaktion unter Scherbedingungen (z.B. EP 0 635 720 A2), verwenden für die Berechnung der Scherrate als Standard z. B. eine Blutviskosität von 3000 $\mu$Pa*s. Unter dieser Bedingung werden dann sämtliche Messungen durchgeführt, ohne zu bewerten, dass erhebliche Viskositätsunterschiede bei unterschiedlichen Patienten bestehen. Nachdem die Viskosität als Formelbestandteil erheblichen Einfluss auf die Größe der Scherrate ausübt, wird dadurch in der Realität mit sehr abweichenden Scherraten gegenüber den vorgegeben gemessen, was zu Verfälschungen der Messergebnisse führt. In vorteilhafter Weise kann bei den zuvor beschriebenen Messeinrichtungen, die Blutviskosität in der Startphase über die genau bemessene Geometrie der Strömungsöffnungen, dem eingestellten Volumenfluss und den daraus resultierenden Förderdruck über den rechnergesteuerten Regelmechanismus bestimmt werden. Somit kann automatisch die richtige Scherrate eingestellt werden und der Viskositätseinfluss weitgehend korrigiert werden, beim weiteren Ablauf der Messung.

**[Beispiele]**

Anhand der Figuren wird an Ausführungsbeispielen die Erfindung noch näher erläutert. Es zeigt:

Figur 1   ein Ausführungsbeispiel einer Messanordnung, zur Erfassung von Gerinnungsfunktionen der globalen, insbesondere der primären Hämostase in Vollblut oder plättchenreichem Plasma.

Fig. 2   eine Ausführungsform für eine Reaktionsöffnung, welche bei der Reaktionseinrichtung gemäß Figur 1 zum Einsatz kommen kann;

Figur 3   graphische Darstellungen von Messergebnissen, die mit dem Ausführungsbeispiel der Messanordnung erreicht werden;

Figur 4   graphische Darstellungen verschiedener möglicher Scherkraft/Scherrate-Kennlinien;

Figur 5   eine graphische Darstellung einer Kennlinie zur Regelung des Volumenflusses in Abhängigkeit vom Druckanstieg in einer Reaktionsöffnung bei konstanter Scherrate.

Figur 6   Messergebnisse von Hämostasefunktionen.

Figur 7   Messergebnisse von Hämostasefunktionen.

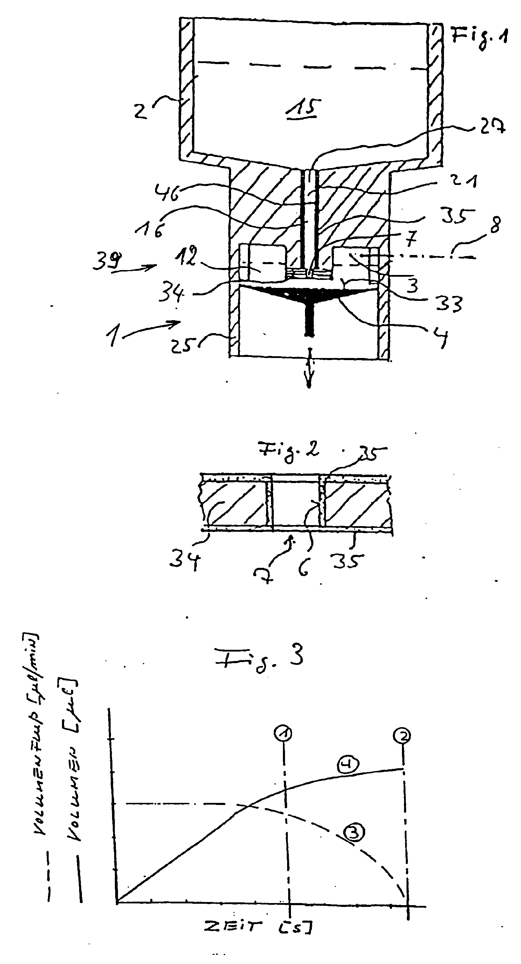

Bei der in Figur 1 dargestellten Vorrichtung ist ein Vorratsraum 15 für zu untersuchendes Blut in einem Vorratsgefäß 2 vorgesehen. Für die Untersuchung, z.B. zur Erfassung der Plättchenfunktion der primären Hämostase und/oder der Funktionseigenschaften der globalen Hämostase, wird das Blut aus dem Vorratsraum 15 durch eine Reaktionseinrichtung 39 transportiert. Die Reaktionseinrichtung 39 besitzt eine Reaktionsöffnung 7.

Die Reaktionsöffnung der Reaktionseinrichtung 39 kann so ausgebildet sein, dass Blutbestandteile, insbesondere Blutplättchen, dort haften bleiben und aggregieren und die Reaktionsöffnung teilweise oder ganz zusetzen. Hierdurch verengt sich der in der Reaktionsöffnung vorgesehene Strömungsquerschnitt und damit steigt der Strömungswiderstand. Ein Förderdruck, welcher dem Druckunterschied zwischen einer von einer Fördereinrichtung erzeugtem Druck, insbesondere Saugdruck, und dem Druck der Außenseite (Atmosphärendruck) entspricht, wirkt auf das zu untersuchende Blut im Vorratsraum 15. Dieser Förderdruck ändert sich bei der Querschnittsverengung der Reaktionsöffnung infolge der möglichen Anlagerung und Aggregation der Thrombozyten bzw. durch eine Verringerung der Fließfähigkeit des Blutes, durch Einsetzen der globalen, insbesondere der primären Blutgerinnung und einem hierbei ansteigendem Strömungswiderstand.

Beim dargestellten Ausführungsbeispiel wird zur Erzeugung des Förderdruckes, der in einem Arbeitsraum 12 an der einen Seite der Reaktionseinrichtung 39 wirkt, eine Kolben-Zylinder-Anordnung 1 verwendet. Diese besitzt einen Zylinder 25 (Messzylinder), in welchem axial ein Kolben 4 verschiebbar geführt ist. Beim dargestellten Ausführungsbeispiel befindet sich ein Druckmessraum 3 unterhalb des Vorratsraumes 15. Der Druckmessraum 3 befindet sich innerhalb eines druckdichten Raumes, in den das zu untersuchende Blut nach dem Durchtritt durch die Reaktionseinrichtung 39 gelangt. Beim dargestellten Ausführungsbeispiel befindet sich dieser druckdichte Raum im Arbeitsraum 12 der Kolben-Zylinder-Anordnung 1. Das durch die Reaktionsöffnung 7 der Reaktionseinrichtung 39 hindurchgetretene Blut sammelt sich auf einer Arbeitsfläche 33, welche bei den dargestellten Ausführungsbeispiel nach oben gerichtet ist. Der Kolben 4 ist gegenüber der Innenwand des Zylinders 25 abgedichtet, so dass die Menge des sich ansammelnden Blutes als Messgröße für die Volumenflussmenge verwendet werden kann. Ein sich an der Zylinderwand bildender Flüssigkeitsmeniskus sorgt für einen zusätzlichen gasdichten Verschluss. Der Zylinder 25 kann somit gleichzeitig als Messzylinder dienen, da die Bewegung des Kolben 4 durch einen Antrieb von einer Steuereinheit geregelt werden kann und so der Volumenstrom und die Volumenflussmenge durch die Reaktionsöffnung, als Messgröße genau erfasst werden kann.

Bei der Ausführungsform der Fig. 1 ist eine Reaktionsöffnung 7 am unteren Ende des in einem Fortsatz des Gefäßbodens verlaufenden Röhrchens 16 vorgesehen. Die Strömungsöffnung 21 des Röhrchens 16 kann als Zufuhröffnung 27 und/oder Scheröffnung 46 wirken. Die Reaktionsöffnung 7 und eine Trennwand 34 sind so ausgebildet, wie es in Figur 2 dargestellt ist. Die Trennwand 34 besteht aus nichtporösem Material, das an einer der beiden Trennwandflächen oder an beiden Trennwandflächen und an der Begrenzungsfläche 6 der Reaktionsöffnung 7 mit einer bioaktiven Beschichtung 35, wie nachstehend beschrieben, versehen ist. Bei der dargestellten Ausführungsform sind beide Trennwandflächen der Trennwand 34 und die Begrenzungsfläche der Öffnung mit einer bioaktiven Beschichtung 35 versehen. Die bioaktive Beschichtung 35 kann hierbei aus Kollagen (auch natürliches rekombinantes Kollagen oder gereinigte Kollagen-Subtypen), synthetischen Peptiden mit kollagenähnlichen Aminosäuresequenzen, Laminin, Fibronektin, Thrombospondin oder anderen bioaktiven Substanzen (US 5,854,076 A oder US 5,662,107 A), an denen Plättchen adherieren oder aus einer Mixtur aus Kollagen (wie oben) oder synthetischen Peptiden mit jeweils Adenosindiphosphat (ADP), Adrenalin, Fibronektin, Laminin, Thrombospondin oder anderen die Plättchen aktivierende Substanzen bestehen.

Bei dem in der Fig. 1 dargestellten Ausführungsbeispiel befindet sich die Reaktionsöffnung 7 am unteren Ende des Röhrchens 16, welches eine Länge von 0 bis etwa 35 mm und einen Durchmesser von etwa 0,150 bis 2 mm haben kann. Die Trennwand 34 mit der Reaktionsöffnung 7, kann auch etwa in der Mitte des Röhrchens 16 vorgesehen sein in Richtung dessen Längsachse (nicht dargestellt). Die Trennwand 34 mit der Reaktionsöffnung 7, kann auch ohne das Röhrchen 16 in einer Ausnehmung des Gefäßbodens angeordnet sein. Der Öffnungsdurchmesser der Reaktionsöffnung 7 kann etwa 0,100 bis 0,500 mm betragen. Die Wanddicke der Trennwand kann etwa 0,10 bis 6 mm betragen.

Mit dem erläuterten Ausführungsbeispiel kann die Erfassung der Plättchenfunktion der primären Hämostase auch in der Weise durchgeführt werden, dass der gemessene Druck durch Rückkopplung auf einen Solldruckwert gehalten wird und als Maß für die Aggregation bzw. Koagulation der Blutplättchen die Blutflussmenge durch die Kapillare bestimmt wird (DE 35 41 057 A1).

Ferner ist es bei den Ausführungsbeispiel möglich, das Erfassungsverfahren so durchzuführen, dass die Druckänderung, die sich während der fortschreitenden Zusetzung des jeweiligen Strömungsweges in der Reaktionseinrichtung 39 ausbildet, in bestimmten Zeitabständen gemessen und der Volumenstrom jeweils so verändert wird, daß er einer vorbestimmten Funktion entspricht. Auch kann während vorbestimmter Zeitabstände der Druck konstant gehalten werden und danach, wenn der Volumenstrom um einen Betrag abgefallen ist, nachgeregelt werden, bis er der vorgegebenen Funktion entspricht (DE 196 17 407 A1).

In bevorzugter Weise kommt ein neues erfindungsgemäßes Verfahren zum Einsatz, bei welchen in Abhängigkeit vom im Druckmessraum 3 gemessenen Druck der Volumenstrom des zu untersuchenden Blutes durch die Reaktionseinrichtung 39 so eingestellt wird, dass eine vorbestimmte Scherraten- bzw. Scherkraftkennlinie eingehalten und vorzugsweise die Scherrate oder Scherkraft konstant gehalten wird.

Für die klinische Bewertung kann hierbei jeweils die Volumenflussmenge und/oder der dann vorhandene Volumenstrom nach Ablauf einer vorgegebenen Messzeit oder bei vorgegebener Volumenflussmenge die abgelaufene Zeit und/oder der dann vorhandene Volumenstrom oder bei vorgegebenem Volumenstrom, die abgelaufene Zeit und/oder die dann vorhandene Volumenflussmenge, verwendet werden. Ebenso kann der Druckanstieg nach einer vorgegebenen Zeit oder die abgelaufene Zeit nach Vorgabe eines Druckanstieges für die Parameterbildung verwendet werden.

Die Einstellung des Volumenstroms erfolgt nach folgender Beziehung:

$$\text{Volumenstrom} \qquad V' = \frac{2\gamma^4 l^3 \eta^3 \pi}{\Delta p^3}$$

Hierin bedeuten:

V' den Volumenstrom des zu untersuchenden Blutes durch die Reaktionseinrichtung, insbesondere durch die Scheröffnung;

$\Delta p$ den im Druckmessraum gemessenen Druck;

l die Länge des Strömungsweges in der Apertur, insbesondere in der Scheröffnung

$\eta$ die Viskosität des zu messenden Blutes;

$\pi$ 3,14.

$\gamma$ die Scherrate

Die Steuerung der Messanordnung, insbesondere der Kolbenbewegung kann so erfolgen, dass eine Blutströmung entlang einer vorbestimmten Scherraten- bzw. Scherkraftkennlinie verläuft.

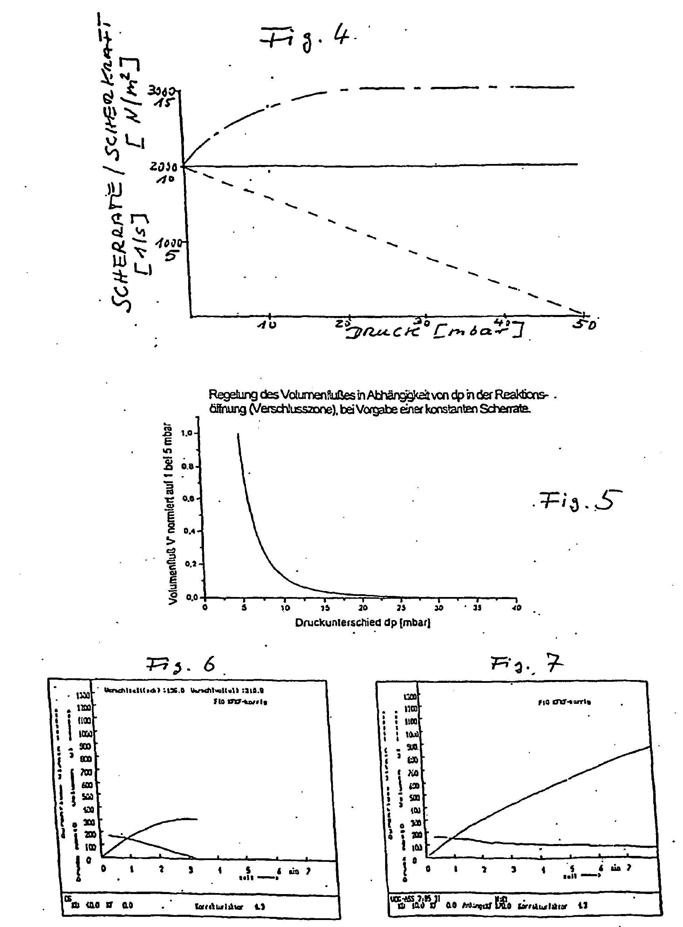

In der Fig. 4 sind in strichpunktierter Form eine nichtlineare ansteigende Scherkraftkennlinie sowie in strichlierter Darstellung eine abfallende lineare Kennlinie dargestellt. Der Verlauf der jeweiligen Kennlinie für die Scherrate (1/s) bzw. der Scherkraft (N/m$^2$), kann gegebenenfalls in Abhängigkeit von der zu erstellenden Diagnose, für welche die Messung durchgeführt wird, gewählt werden. In bevorzugter Weise wird eine konstante Kennlinie (durchgezogene Linie in der Fig. 26) für eine bestimmte Scherrate bzw. Scherkraft gewählt. Bei ansteigendem Fließdruck, beispielsweise durch Anlagerung bzw. Aggregation der Blutplättchen in der Reaktionsöffnung, wird die gewünschte Kennlinie durch Steuerung der Kolbenbewegung entsprechend der oben angegebenen Formel erreicht.

Die Fig. 3 zeigt parameterbildende Größen, mit der strichpunktierten Linie 1 als Zeitbegrenzung für die gestrichelte Linie 3 für den Volumenstrom und die durchgezogene Linie 4 für die Volumenflussmenge, deren durch die Zeitbegrenzung ermittelte Werte Messparameter bilden oder wenn die gestrichelte Linie 3 für den Volumenstrom gegen Null geht, sich als Messparameter die Messzeit, gekennzeichnet durch die Linie 2 ergibt sowie der Wert für die Volumenflussmenge der durchgezogenen Linie 4 oder die durchgezogene Linie 4 für die Volumenflussmenge, sobald deren vorgegebener Wert erreicht ist, die Zeit der strichpunktierten Linie 1 bildet. Diese Parameter werden gebildet durch Gerinnungsreaktionen der globalen, insbesondere der primären Hämostase, welche u.a. unter Einwirkung einer Schergröße in der Reaktionsöffnung(en) entstehen. Dabei folgt die Schergröße einer vorgegebenen Kennlinie. Diese beschriebenen Parameter können im Ausführungsbeispiel gebildet werden.

Die Figur 5, zeigt den Volumenfluss normiert auf 1 bei 5 mbar in Abhängigkeit vom Druckunterschied dp für die Regelung des Volumenflusses in Abhängigkeit von dp in der Reaktionsöffnung bei Vorgabe einer konstanten Scherrate.

Die Fig. 6 und Fig. 7 zeigen Messergebnisse einschließlich der Druckverlaufskurve bei Überprüfung der Gerinnungsfunktion der globalen, insbesondere der primären Hämostase bei Regelung des Volumenflusses in Abhängigkeit der Druckänderung in der Reaktionsöffnung, bei Vorgabe einer konstanten Scherrate gemäß Fig. 5. Messwerte für die klinische Auswertung, sind die Verschlusszeit und das Verschlussvolumen. Bei Fig. 6 geht der Volumenstrom in 196 Sekunden und einer Volumenflussmenge von 310,9 $\mu$l zu Null.

Bei Fig. 7 kommt es kaum zu einer Anlagerung von Plättchen in der Reaktionsöffnung durch den plättchenhemmenden Einfluss von ASS (Acetylsalicylsäure). Die Messgrenzen werden wegen des medikamentösen Einflusses auf die Plättchenfunktion nicht erreicht.

**Patentansprüche**

1. Vorrichtung zur Untersuchung von Eigenschaften der globalen, insbesondere der primären Hämostasefunktionen in Vollblut oder plättchenreichem Plasma mit einem Vorratsraum (15) für das zu untersuchende Blut, einer Reaktionseinrichtung (39), die wenigstens eine Reaktionsöffnung (7) in einer Trennwand (34) aufweist, durch welche zur Durchführung bestimmter Reaktionen zu untersuchendes Blut transportiert wird, einer Fördereinrichtung (1) für den Transport des Blutes durch die Reaktionseinrichtung (39) und einem Blutsammelraum (10) zum Auffangen des durch die Reaktionseinrichtung (39) transportierten Blutes,
   **dadurch gekennzeichnet, dass** die Trennwand (34) aus nichtporösem Material besteht und die wenigstens eine Reaktionsöffnung (7) an ihrer Begrenzungsfläche (6) eine bioaktive Beschichtung aufweist.

2. Vorrichtung nach Anspruch 1,
   **dadurch gekennzeichnet, dass** ein Öffnungsdurchmesser der Reaktionsöffnung (7) etwa 0,100 bis 0,500 mm beträgt.

3. Vorrichtung nach Anspruch 1 oder 2,
   **dadurch gekennzeichet, dass** im Bereich der Reaktionsöffnung bzw. Reaktionsfläche eine Beschichtung aus Erythrozyten und/oder Leukozyten, insbesondere der Blutgruppe 0 und/oder von-Willebrand-Faktor vorgesehen ist.

4. Vorrichtung nach Anspruch 1,
   **dadurch gekennzeichnet, dass** die Trennwand (34) an einer oder an beiden Trennwandflächen mit einer bioaktiven Beschichtung (35) versehen ist.

5. Verfahren zur Erfassung der globalen Hämostasefunktion, insbesondere der primären Hämostase, bei welchem mit einer Vorrichtung gemäß einem der Ansprüche 1 bis 4 aus einem Vorratsraum zu untersuchendes Blut unter vorgegebenen Fließbedingungen durch wenigstens eine Reaktionsöffnung einer Reaktionseinrichtung gefördert wird und beim Anlagern von Blutbestandteilen an den Reaktionsflächen unter Einwirkung von Scherkräften in der wenigstens einen Reaktionsöffnung ein sich ändernder Druck gemessen wird,
   **dadurch gekennzeichet, dass** in Abhängigkeit vom jeweils gemessenen Druck der Volumenstrom des durch die Reaktionseinrichtung geförderten Blutes so eingestellt wird, dass die Scherrate bzw. die Scherkraft, welche in wenigstens einer Reaktionsöffnung wirksam ist, einer vorgegebenen Kennlinie der Scherrate bzw. Scherkraft folgt.

6. Verfahren nach Anspruch 5,
   **dadurch gekennzeichnet, dass** die Scherrate bzw. die Scherkraft konstant gehalten wird.

7. Verfahren nach Anspruch 5 oder 6,
   **dadurch gekennzeichnet, dass** vor Beginn der Messung die Viskosität des Blutes bestimmt wird und in Abhängigkeit davon, die Scherrate bzw. Scherkraft eingestellt wird.

8. Verfahren nach Anspruch 5 und 7,
   **dadurch gekennzeichnet, dass** die Viskositätsmessung in der Reaktionseinrichtung durchgerührt wird.

9. Verfahren zur Erfassung der globalen Hämostasefunktion, insbesondere der primären Hämostase nach einem der Ansprüche 5 bis 8,
   **dadurch gekennzeichnet, dass** die Reaktionseinrichtung gemäß Anspruch 1 oder 2 als Einmalteil verwendet wird.

**Claims**

1. A device for examining properties of global, in particular primary, hemostatic functions in whole blood or platelet-rich plasma, comprising a storage chamber (15) for the blood to be examined, a reaction device (39) having at least one reaction opening (7) in a dividing wall (34) through which blood to be examined is transported for carrying out certain reactions, a conveying means (1) for transporting the blood through the reaction device (39), and a blood collection chamber (10) for collecting the blood transported through the reaction device (39),
   **characterized in that** said dividing wall (34) is made of a non-porous material, and said at least one reaction opening (7) has a bioactive coating on its boundary surface (6).

2. A device according to claim 1,

**characterized in that** an opening diameter of the reaction opening (7) is about 0.100 to 0.500 mm.

3. A device according to claim 1 or 2,
**characterized in that** a coating of erythrocytes and/or leukocytes, in particular of blood group 0 and/or of von-Willebrand factor, is provided in the area of the reaction opening or reaction surface.

4. A device according to claim 1,
**characterized in that** the dividing wall (34) is provided with a bioactive coating (35) on one or both of its surfaces.

5. A method of analysing the global hemostatic function, in particular the primary hemostasis, in which, using a device as defined in any of claims 1 to 4, blood to be examined is conveyed under defined flow conditions from a storage chamber through at least one reaction opening of a reaction device, and a varying pressure is measured when blood components deposit on the reaction surfaces under the effect of shear forces in the at least one reaction opening,
**characterized in that,** depending on the currently measured pressure, the volume flow of the blood conveyed through the reaction device is adjusted such that the shear rate or shear force acting in at least one reaction opening follows a predetermined characteristic of the shear rate or shear force.

6. A method according to claim 5,
**characterized in that** the shear rate or shear force is maintained constant.

7. A method according to claim 5 or 6,
**characterized in that,** prior to measurement, the viscosity of the blood is determined, and **in that** the shear rate or shear force is adjusted depending on viscosity.

8. A method according to claims 5 and 7,
**characterized in that** viscosity measurement is carried out in the reaction device.

9. A method of analysing the global hemostatic function, in particular the primary hemostasis, according to any of claims 5 to 8,
**characterized in that** the reaction device according to claims 1 or 2 is used as a disposable device.


**Revendications**

1. Dispositif pour examiner des propriétés des fonctions de l'hémostase globale, et notamment de l'hémostase primaire, dans du sang complet ou du plasma riche en plaquettes, comprenant un réservoir (15) pour le sang à examiner, un dispositif de réaction (39) ayant au moins un orifice de réaction (7) dans une paroi de séparation (34) à travers laquelle du sang à examiner est transporté afin d'effectuer certaines réactions, un dispositif de transport (1) pour transporter le sang à travers le dispositif de réaction (39) et une chambre de collecte de sang (10) pour recueillir le sang transporté à travers le dispositif de réaction (39),
**caractérisé en ce que** la paroi de séparation (34) est constituée par un matériau non-poreux et que l'au moins un orifice de réaction (7) comporte un revêtement bioactif sur sa périphérie (6).

2. Dispositif selon la revendication 1,
**caractérisé en ce qu'**un diamètre de l'orifice de réaction (7) mesure environ 0,100 à 0,500 mm.

3. Dispositif selon la revendication 1 ou 2,
**caractérisé en ce qu'**une couche d'érythrocytes et/ou de leucocytes, notamment du groupe sanguin 0 et/ou de facteur von Willebrand est prévue dans la région de l'orifice de réaction et/ou de la surface de réaction.

4. Dispositif selon la revendication 1,
**caractérisé en ce que** la paroi de séparation (34) est pourvue d'un revêtement bioactif (35) sur ses deux faces.

5. Procédé pour détecter la fonction de l'hémostase globale, notamment de l'hémostase primaire, dans lequel le sang à examiner est transporté, à l'aide d'un dispositif comme défini dans l'une des revendications 1 à 4, dans des conditions d'écoulement prédéterminées, d'un réservoir à travers au moins un orifice de réaction d'un dispositif de réaction et dans lequel une pression variable est mesurée lors du dépôt de composants sanguins sur les surfaces de réaction sous l'effet de forces de cisaillement dans l'au moins un orifice de réaction,

**caractérisé en ce que** le volume d'écoulement du sang transporté à travers le dispositif de réaction est réglé en fonction de la pression mesurée chaque fois de sorte que la vitesse et/ou la force de cisaillement présente dans au moins un orifice de réaction suive une caractéristique prédéterminée de la vitesse et/ou la force de cisaillement.

**6.** Procédé selon la revendication 5,
**caractérisé en ce que** la vitesse et/ou la force de cisaillement est maintenue constante.

**7.** Procédé selon la revendication 5 ou 6,
**caractérisé en ce que** la viscosité du sang est déterminée avant la mesure et que la vitesse et/ou la force de cisaillement est réglée en fonction de la viscosité.

**8.** Procédé selon la revendication 5 et 7,
**caractérisé en ce que** la viscosité est mesurée dans le dispositif de réaction.

**9.** Procédé pour détecter la fonction hémostatique globale, notamment l'hémostase primaire, selon l'une des revendications 5 à 8,
**caractérisé en ce que** le dispositif de réaction selon la revendication 1 ou 2 utilisé est un dispositif à jeter.

Fig. 1

Fig. 2

Fig. 3

Fig. 4

Regelung des Volumenflußes in Abhängigkeit von dp in der Reaktionsöffnung (Verschlusszone), bei Vorgabe einer konstanten Scherrate.

Fig. 5

Fig. 6

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19617407 A1
- EP 0635720 A1
- EP 0138190 B1
- US 5662107 A
- EP 0316599 A2
- EP 0111942 A
- US 5854067 A
- DE 3541057 A1
- EP 0635720 A2
- US 5854076 A